# EUROPEAN PATENT APPLICATION

(11) **EP 3 173 110 A1**
(43) Date of publication of application: **31.05.2017**
(21) Application number: 15197099.3
(22) Date of filing: 30.11.2015
(51) Int. Cl.: A61M 1/10

(54) **ROTARY PUMP SUSPENSION SYSTEM ARRANGEMENT, ESPECIALLY OF IMPLANTABLE CENTRIFUGAL HEART ASSIST PUMP**

(71) Applicant: Fundacja Rozwoju Kardiochirurgii Im. Prof. Zbigniewa Religi, 41-800 Zabrze (PL)
(72) Inventor: ALTYNTSEV, Ievgenii, 40-024 Sumy (UA); DARLAK, Maciej, 41-710 Ruda l ska (PL); KUSTOSZ, Roman, 41-800 Zabrze (PL)
(74) Representative: Caban, Wlodzimierz

(57) **Abstract**

The present invention relates to a suspension assembly of a centrifugal pump impeller, in particular of an implantable ventricular assist centrifugal pump, applied in cardiologic therapy.

According to the present invention a lower magnetic bearing (5,6, 13,14) of the magnetic bearing assembly inside an inflow cannula (12) mounted in the pump housing (2) has a first set of two cylindrical magnets (13, 14), the position of said cylindrical bearings being preferably adjusted by screwing and/or unscrewing of a holder (7) of the inflow cannula (12) with respect to the pump housing (2), whereas channels (9a, 9b) of the hydrodynamic bearing assembly are tapered in the direction of the main channel (19) of the pump in the direction of the internal surface of the shroud (17) and the inlet channel (20) of the pump, that is in the direction opposite to the direction of rotation of the impeller (1).

## Description

The present invention relates to a suspension assembly of a centrifugal pump impeller, in particular to an implantable ventricular assist centrifugal pump.

There are numerous strict technical requirements which must be met by centrifugal pumps for pumping blood in ventricular assist devices. The main requirement for said pumps is to ensure such flow of blood that shear stresses affecting blood cells is reduced to a minimum and the time during which morphological elements of blood are in the space where they are affected by increased shear stress e.g. in the area of the impeller bearing is also reduced to a minimum. At the same time a centrifugal pump provided for this purpose must minimise or even eliminate the mechanical friction of the rotating elements.

For example, EP 2 405 141 A1 discloses a pump comprising a suspension system consisting of an active and passive magnetic suspension. In order to balance all forces affecting the impeller, additional solenoids have been placed in the pump housing, said solenoids generating appropriate electromagnetic field and therefore enable balancing all forces affecting the impeller of the pump.

WO 2013/134319 A1 discloses a suspension system of an impeller consisting of two types of bearings: a hydrodynamic bearing in the upper part of the impeller and a passive magnetic bearing. Said magnetic bearing enables balancing the radial forces affecting the impeller, whereas the hydrodynamic bearing ensures compensation of axial forces. The magnetic bearing generates an auxiliary magnetic force which induces the movement of the impeller along the axis thereof in the direction of the pump's inlet opening. The hydrodynamic bearing counterbalances this force.

Moreover, WO 2007/084339 A2 discloses a solution wherein the impeller suspension consists of three elements: a hydrodynamic bearing and permanent magnets in the impeller and in the pump housing, as well as electric magnetic elements in the pump housing. The impeller is affected by: the force generated by the permanent magnets located in the upper shroud of the impeller and in the upper housing of the pump; the force acting between the permanent magnets located in the lower shroud of the impeller and solenoids mounted in the magnetic core in the lower pump housing; the force exerted by magnetic bearings located on the internal - upper and lower - surface of the pump housing. The magnetic forces counterbalance each other in the geometric centre of the pump interior. The hydrodynamic bearings produce the hydrodynamic force which maintains the balance of the impeller and counterbalances the magnetic forces.

Centrifugal pumps ensuring contactless work of the impeller within the centrifugal pump housing are also known in the prior art where magnetic forces are used, as is, for example, disclosed in US 6 015 434 or JP 2010158532.

EP 2405141 or CN 2012 30980, on the other hand, disclose a use of magnetic elements with a hydrodynamic bearing.

The aim of the present invention is to provide such impeller suspension assembly of a centrifugal pump, in particular an implantable ventricular assist centrifugal pump that, by meeting the requirement of contactless work of the impeller within the pump housing, ensures elimination of shear stresses affecting blood cells and reduces the time during which blood cells are present in the areas with increased shear stresses in the proximity of the hydrodynamic bearing assembly to a minimum.

The essence of the invention is that the lower bearing has an inflow cannula mounted in the pump housing, wherein the inflow cannula is equipped with a set of two cylindrical magnets slidable along the axis of rotation of the pump, and the channels of the hydrodynamic bearing assembly are tapered in the direction of the internal surface of the shroud, towards the area of the inlet channel and in the direction opposite to the direction of rotation of the impeller.

It is preferable that the inflow cannula have a threaded holder for the cylindrical magnets, screwed into the pump housing, which allows to accurately position of the magnets.

The main advantage of the assembly according to the invention is that it is possible to set the centre of balance of all applied magnetics forces precisely in the middle of the distance between the upper and the lower wall of the pump housing, which is practically achieved by screwing or unscrewing the magnets in the inflow cannula. Owing to this the stabilising forces are increased, which allows to adjust the point of counterbalance of the magnetic forces acting upon the impeller from the side of the magnetic bearings and from the side of the impeller, while simultaneously ensuring symmetrical action of the magnetic forces with respect to the geometrical centre of the pump interior and minimising the value of the magnetic forces acting near the walls of the pump housing. The shape of the channel helices ensures smooth and fast movement of blood cells inside the pump in the area of the hydrodynamic bearing. As a whole the assembly according to the invention allows to maintain the impeller at a greater distance from the housing walls, with lower hydrodynamic forces counterbalancing the magnetic forces, owing to which shear stresses generated in the hydrodynamic bearing are decreased and the distance of counterbalancing of all forces acting upon the pump impeller is increased further from the middle of the distance between the internal parts of the pump housing, which allows to maintain the necessary driving power of the pump at the previous level.

The invention has been presented in greater detail in the embodiment shown in a drawing, where fig. 1 is a simplified cross section of the centrifugal pump, fig. 2 is the assembly of permanent magnets of the impeller suspension, fig. 3 is a fragment of the inflow cannula complete with the possibility to adjust the position of the set of cylindrical magnets, fig. 4 is a simplified element of the hydrodynamic bearing assembly, fig. 5 is a blade of the hydrodynamic bearing.

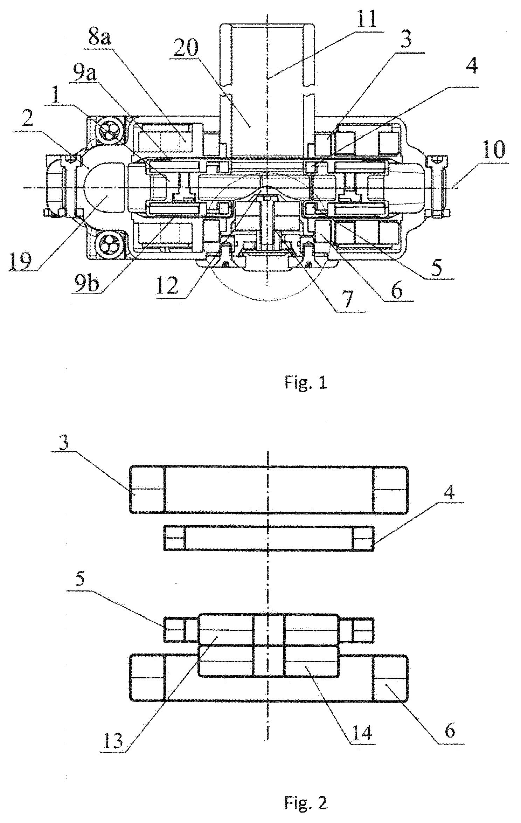

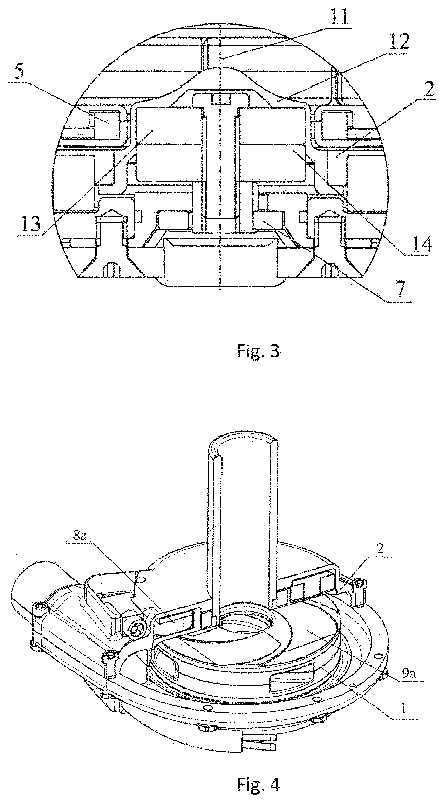

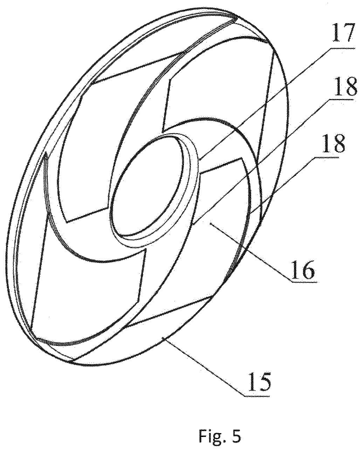

The centrifugal pump according to the invention (fig. 1) has an impeller 1 suspended in a contactless manner within the pump housing 2 in a hybrid system of a stationary magnetic bearing with permanent magnets 3, 4 and 5, 6 and 7 and a dynamic hydraulic bearing 9a and 9b cooperating such that the action of magnetic forces is counterbalanced in the geometrical axis of the impeller flow channel 10, perpendicular to the axis of rotation 11 of said impeller. Counterbalancing of the magnetic forces is ensured by a system of two ring magnets 13, 14 (fig. 2), located on an adjustable holder 7 (fig. 1, fig. 3) and cooperating with magnets of the lower magnetic bearing 5, 6 in the pump housing and in the lower shroud of the pump impeller. Adjustment of the position of the assembly of magnets in the inflow cannula 13, 14 with respect to the other magnets of the lower magnetic bearing 5, 6 allows to counterbalance all magnetic forces, including the magnetic forces generated in the upper magnetic bearing 3, 4 on the central plane of the impeller 10. The hydrodynamic bearing 9a and 9b symmetrically located on two opposite shrouds of the impeller 1: the upper shroud 9a and the lower shroud 9b ensures that the impeller's movement is monitored and said impeller is prevented from moving towards the pump housing 2. Each of said two hydrodynamic bearings consists of four helical channels (fig. 5), said channels consisting of: a channel inlet 15, the part for blood impounding 16, an edge of the impounding part 18 and a helical channel outlet 17 of the hydrodynamic bearing. It is characteristic for the present design that the inlet to the helical blades of the hydrodynamic bearing 15 is supplied from the main channel 19 of the pump, whereas the outlet 17 from the helical blades of the hydrodynamic bearing is directed towards the outlet channel 20 of the pump.

## Claims

1. A suspension assembly of a centrifugal pump impeller, in particular of an implantable ventricular assist centrifugal pump, wherein for adjusting the position of the impeller with respect to the pump housing in the direction parallel to the axis of rotation of the impeller a magnetic bearing assembly is used, and said magnetic bearing is based on ring magnets ensuring a two-point support of the impeller axis, said ring magnets being mounted in an upper bearing in the upper pump housing by an inlet channel and in un upper shroud of the impeller and a lower bearing in the lower pump housing and in a lower shroud of the impeller, wherein adjustment of the position of the impeller with respect to the housing in the direction parallel to the axis of rotation of the impeller is achieved by means of hydrodynamic bearings consisting of two sets of construction elements located symmetrically with respect to the central plane of the impeller, parallel to the inlet channel, said construction elements being located on the upper shroud and on the lower shroud of the impeller, wherein each of said bearings provides axially symmetrical helical channels, **characterised in that** the lower magnetic bearing (5, 6, 13, 14) inside an inflow cannula (12) mounted in the pump housing (2) has a set of two cylindrical magnets (13, 14) slidable along the axis of rotation (11) of the pump impeller (1), and channels (9a, 9b) of the hydrodynamic bearing assembly are tapered from the side of the main channel (19) of the pump in the direction of the internal surface of the shroud and the inlet channel (20) of the pump, in the direction opposite to the direction of rotation of the impeller (1).

2. The assembly according to claim 1, **characterised in that** the inflow cannula (12) has a threaded holder (7) screwed into the pump housing (2).

3. The assembly according to claim 1, **characterised in that** each channel (9a, 9b) of the hydrodynamic bearing is of a helical shape, and the inlet (15) of the channel (9a) is located in the main channel (19) of the pump, whereas the outlet (17) of the channel (9a) is directed directly to the inlet channel (20) of the impeller (1).
